# EUROPÄISCHE PATENTSCHRIFT

(11) **EP 2 376 426 B1**
(45) Veröffentlichungstag und Bekanntmachung des Hinweises auf die Patenterteilung: **24.09.2014**
(21) Anmeldenummer: 09764823.2
(22) Anmeldetag: 04.12.2009
(51) Int. Cl.: C07C 231/12, C07C 233/03

(54) **VERFAHREN ZUR HERSTELLUNG VON N-VINYLCARBONSÄUREAMIDEN**
METHOD FOR PRODUCING N-VINYLCARBOXAMIDES
PROCÉDÉ DE FABRICATION D'AMIDES D'ACIDES N-VINYLCARBOXYLIQUES

(30) Priorität: 15.12.2008 EP 08171677
(43) Veröffentlichungstag der Anmeldung: 19.10.2011
(73) Patentinhaber: BASF SE, 67056 Ludwigshafen (DE)
(72) Erfinder: WINTER, Manfred, 67596 Dittelsheim-Heßloch (DE); WEIGL, Hagen, 68526 Ladenburg (DE); KRAMER, Andreas, 67159 Friedelsheim (DE)
(74) Vertreter: Peatfield, Jeremy William
(86) Internationale Anmeldenummer: PCT/EP2009/066387
(87) Internationale Veröffentlichungsnummer: WO 2010/072543

(56) Entgegenhaltungen:
- DE-A1- 1 668 038
- DE-B- 1 224 304
- P. KURTZ ET.AL.: "Enamide" LIEBIGS ANN. CHEM., Bd. 764, 1972, Seiten 69-93, XP002583721

## Beschreibung

Die Erfindung betrifft ein Verfahren zur Herstellung von N-Vinylcarbonsäureamiden durch Pyrolyse einer Verbindung der Formel 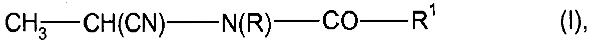 in der R und R¹ für H oder C₁- bis C₆-Alkyl stehen,

in Gegenwart von Feststoffen, die mit Alkalimetall- oder Erdalkalimetallionen dotiert sind, unter vermindertem Druck bei einer Temperatur von 330 bis 750 °C unter Abspaltung von Cyanwasserstoff, Abkühlen, Trennen und Isolieren der Reaktionsprodukte.

Aus der DE-C 1 224 304 und Liebigs Ann. d. Chem. 764, 69-93 (1972) ist ein Verfahren zur Herstellung von N-Vinylcarbonsäureamiden wie N-Vinylformamid oder N-Vinylacetamid durch Pyrolyse von Verbindungen der Formel I bei einer Temperatur in dem Bereich von 250 bis 750 °C unter Normaldruck oder unter vermindertem Druck in einem Rohrreaktor unter Abspaltung von Cyanwasserstoff, Abkühlen, Trennen und Isolieren der Reaktionsprodukte bekannt. Der Rohrreaktor enthält zur Vergrößerung der Reaktionsoberfläche Feststoffe wie Quarzscherben, Glas, Eisen, Siliciumcarbid, Aluminiumfluorid, Koks, Calciumphosphat oder Tonerde. Der bei der Reaktion entstehende Cyanwasserstoff wird nach dem Abtrennen der Vinylcarbonsäureamide kondensiert.

Aus der EP-A 0 184 694 ist ein Verfahren zur Herstellung von Umsetzungsprodukten des Cyanwasserstoffs mit Basen oder Carbonylverbindungen bekannt, wobei der Cyanwasserstoff durch Pyrolyse von Formamid oder N-Acylderivaten des 1-Amino-1-cyanethans oder deren Substitutionsprodukten bei 250 bis 650 °C an Feststoffen als Katalysator unter einem Druck von 5 bis 200 mbar hergestellt wird. Die Pyrolysegase werden unter vermindertem Druck auf eine Temperatur in dem Bereich von 200 bis-10 °C abgekühlt. Dabei kondensieren die bei der Pyrolyse gebildeten Monomeren und gegebenenfalls nicht umgesetzte Einsatzstoffe. Das Kondensat enthält lediglich geringe Mengen Cyanwasserstoff. Die Hauptmenge des Cyanwasserstoffs wird unter vermindertem Druck einer Chemisorption mit Basen oder Carbonylverbindungen unterworfen und in Form der Kondensationsprodukte ausgeschleust und auf Atmosphärendruck entspannt. Bei diesem Verfahren wird die Handhabung größerer Mengen an Cyanwasserstoff vermieden.

Aus der EP-A 0 184 074 ist ein Verfahren zur Herstellung von N-Vinylformamid bekannt, wobei man Formylalaninnitril in Gegenwart von Feststoffen als Katalysator unter vermindertem Druck bei einer Temperatur in dem Bereich von 250 bis 650 °C pyrolysiert. Als Katalysator kann man diejenigen Feststoffe verwenden, die in der obengenannten DE-C 1 224 304 angegeben sind sowie Alkalimetall- und Erdalkalimetallcarbonate, Magnesiumoxid, Calciumoxid, Bariumoxid sowie insbesondere solche Katalysatoren, die Alkalimetall- oder Erdalkalimetallcarbonate auf α-Aluminiumoxid enthalten. Diese Katalysatoren werden hergestellt, indem man α-Aluminiumoxid mit wasserlöslichen Salzen wie Calciumacetat tränkt und einer thermischen Behandlung unterwirft. Die Pyrolyseprodukte bestehen im Wesentlichen aus N-Vinylformamid, Cyanwasserstoff und geringen Mengen an nicht umgesetztem Formylalaninnitril. Sie werden unter einem Druck von 10 bis 150 mbar auf eine Temperatur von 200 bis -10 °C abgekühlt, so dass eine weitgehende Trennung in N-Vinylformamid und Cyanwasserstoff erfolgt, das Monomere wird aus dem Kondensat isoliert und der nicht kondensierte Cyanwasserstoff unter einem Druck von 5 bis 140 mbar in etwa molarem Verhältnis einer Chemisorption mit Acetaldehyd bei Temperaturen von -20 bis 30 °C unter Bildung von Milchsäurenitril unterworfen und nach dem Druckausgleich mit der Atmosphäre isoliert. Bei diesem Verfahren wird ebenfalls die Bildung größerer Mengen an freiem Cyanwasserstoff vermieden, weil er direkt zu einem Produkt umgesetzt wird, das gefahrlos gehandhabt werden kann.

Bei der praktischen Durchführung von Verfahren zur Herstellung von N-Vinylcarbonsäureamiden durch Pyrolyse kommt es mit zunehmender Produktionsdauer zu einem Abfall von Umsatz und Selektivität. Der Katalysator muss dann erneuert bzw. reaktiviert werden. Bei Rohrreaktoren verursacht der Austausch des Katalysators einen erheblichen Aufwand, weil solche Reaktoren sehr viele, meistens mehrere hundert Rohre aufweisen, die dann entleert und wieder befüllt werden müssen. Beim Einbau und beim Ausbau des Katalysators ist darüber hinaus immer mit einer mechanischen Beschädigung des Katalysators zu rechnen. Um von einem Katalysator kohlenstoffhaltige Ablagerungen zu entfernen, kann man ihn zwar innerhalb des Reaktors mit einem Sauerstoff enthaltendem Gas bei höheren Temperaturen behandeln. Dadurch lässt sich jedoch nicht beliebig oft die volle Wirksamkeit des Katalysators wieder herstellen. Es ist dann vielmehr notwendig, den Katalysator auszubauen und ihn außerhalb des Reaktors durch Behandlung mit einer Alkalimetall- und/oder Erdalkalimetallbase zu reaktivieren und einer thermischen Behandlung zu unterwerfen.

Der Erfindung liegt die Aufgabe zugrunde, ein Verfahren zur Herstellung von N-Vinylcarbonsäureamiden durch Pyrolyse zur Verfügung zu stellen, wobei die Aktivierung und Reaktivierung des Katalysators im Reaktor vorgenommen werden kann, in dem die Pyrolyse erfolgt.

Die Aufgabe wird erfindungsgemäß gelöst mit einem Verfahren zur Herstellung von N-Vinylcarbonsäureamiden durch Pyrolyse einer Verbindung der Formel 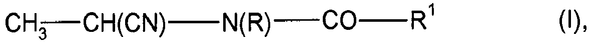 in der R und R¹ für H oder C₁- bis C₆-Alkyl stehen,

in Gegenwart von Feststoffen, die mit Alkalimetall- oder Erdalkalimetallionen dotiert sind, unter vermindertem Druck bei einer Temperatur von 330 bis 750 °C unter Abspaltung von Cyanwasserstoff, Abkühlen, Trennen und Isolieren der Reaktionsprodukte, wenn man einen in einem Rohrreaktor angeordneten Feststoff bei einer Temperatur in dem Bereich von 0 bis 250 °C mit einer Lösung einer Alkalimetall- und/oder Erdalkalimetallbase behandelt, die Lösung ablässt, das restliche Lösemittel, das dem so erhältlichen Katalysator anhaftet, verdampft und ihn danach zur Aktivierung auf eine Temperatur von mindestens 380 °C erhitzt.

Die Verbindungen der Formel I sind beispielsweise aus der zum Stand der Technik genannten EP-A 0 184 694 bekannt, vgl. Spalte 2, Zeile 40 bis Spalte 3, Zeile 23. Vorzugsweise wird als Verbindung der Formel I Formylalaninnitril (Formel I mit R = H und R¹ = H) eingesetzt, um daraus durch Pyrolse N-Vinylformamid herzustellen. Von Interesse sind außerdem solche Verbindungen der Formel I, worin
R = H und R¹ = CH₃ ist, zur Herstellung von N-Vinylacetamid,
R = H und R¹ = C₂H₅ zur Herstellung von N-Vinylpropylamid,
R = CH₃ und R¹ = H zur Herstellung von N-Vinyl-N-methylformamid und
R = CH₃ und R¹ = CH₃ zur Herstellung von N-Vinyl-N-methylacetamid bedeutet.

Die Pyrolyse erfolgt in Gegenwart von Feststoffen, die mit Alkalimetall- und/oder Erdalkalimetallionen dotiert sind. Geeignete Feststoffe sind z.B. in der DE-C 1 224 304, Spalte 2, Zeilen 16 bis 24 sowie in der EP-A 0 184 074, Spalte 1, Zeilen 52 bis 66 angegeben. Beispielsweise eignen sich Siliciumdioxyd, Siliciumcarbid, Aktivkohle, Calciumphosphat, Tonerde, Titandioxid und Aluminiumoxide. Ein besonders bevorzugt eingesetzter Feststoff ist α-Aluminiumoxid. Prinzipiell eignen sich alle porösen Feststoffe als Trägermaterial für den Katalysator. Die Feststoffe werden beispielsweise in Form von Strängen, Kugeln, Röhren, Formkörper oder Plättchen im Rohrreaktor angeordnet.

Der Rohrreaktor kann aus einem einzigen Rohr oder aus einer Vielzahl von Rohren bestehen. Solche Reaktoren können beispielsweise 1 bis 35 000 Rohre, meistens 5 bis 10 000 Rohre, insbesondere bis maximal 3 000 oder auch bis 5 000 Rohre aufweisen, wobei der Rohrdurchmesser in einem Bereich von 0,3 cm bis 25 cm, meistens zwischen 1 cm und 14 cm variieren kann. Der Rohrreaktor kann elektrisch, mit überhitztem Wasserdampf, Kreis- oder Wälzgas oder mit Hilfe eines Salzbades beheizt werden.

Damit die Feststoffe mechanisch möglichst wenig beansprucht werden, erfolgt die Behandlung mit Alkalimetall- und/oder Erdalkalimetallbasen erst nach Einbau der Feststoffe in den Rohrreaktor. Sie werden zu diesem Zweck bei einer Temperatur in dem Bereich von 0 bis 250 °C mit einer Lösung einer Alkalimetall- und/oder Erdalkalimetallbase in Kontakt gebracht. Vorzugsweise verwendet man hierfür wässrige Lösungen. Man kann jedoch auch als Lösemittel für die Basen mit Wasser mischbare Produkte wie Dimethylformamid, Tetrahydrofuran, Dioxan, Methanol, Ethanol, Propanol, Isopropanol, Butanol, Cyclohexanol, Alkylenglykole, Polyalkylenglykole, alkoxylierte Alkohole, alkoxylierte Amine sowie deren Mischungen untereinander oder mit Wasser verwenden. Beispiele für Alkylenglykole sind Ethylenglykol, Propylenglykol-1,2, Propylenglykol-1,3, Butylenglykol-1,2, Butylenglykol-1,4, und Neopentylglykol. Weitere geeignete Lösemittel sind beispielsweise Polyalkylenglykole wie Diethylenglykol, Triethylenglykol, Tetraethylenglykol sowie Ethoxylierungsprodukte von mehrwertigen Alkoholen wie Propylenglykol, Glycerin, Pentaerythrit oder Trimethylolpropan. Der Ethoxylierungsgrad wird vorzugsweise so gewählt, dass die entstehenden Ethoxylierungsprodukte einen Siedepunkt von höchstens 350 °C bei einem Druck von 50 mbar haben. Sofern der im Reaktor angeordnete Katalysator eine Temperatur aufweist, die oberhalb der Siedetemperatur des Lösemittels liegt, erfolgt die Behandlung des Katalysators mit Lösungen von Basen unter erhöhtem Druck. Hierbei handelt es sich beispielsweise um den Druck, der sich bei der jeweiligen Temperatur einstellt. Vorzugsweise erfolgt die Behandlung des Katalysators bei einer Temperatur von 10 bis 80 °C mit einer wässrigen Lösung einer Alkalimetall- und/oder einer Erdalkalimetallbase unter Atmosphärendruck.

Als Alkalimetall- und/oder Erdalkalimetallbasen kommen beispielsweise alle wasserlöslichen Salze und Basen von Alkali- und Erdalkalimetallen in Betracht wie insbesondere Natriumhydroxid, Kaliumhydroxid, Calciumhydroxid, Strontiumhydroxid, Bariumhydroxid, Calciumacetat, Calciumformiat, Bariumformiat, Magnesiumacetat, Natriumhydrogencarbonat, Kaliumhydrogencarbonat, Natriumcarbonat, Kaliumcarbonat, Natriumacetat und Kaliumäcetat. Die wässrigen Lösungen, mit denen die Feststoffe im Reaktor behandelt werden, haben beispielsweise eine Konzentration von mindestens 5 Gew.-% einer Base oder eines Salzes bis zur jeweiligen Sättigungskonzentration. Die Konzentration einer Base oder eines Salzes in der wässrigen Behandlungslösung beträgt meistens 1 bis 50, insbesondere 30 bis 45 Gew.-%.

Nach der Behandlung der Feststoffe mit einer Lösung einer Alkalimetall- und/oder Erdalkalimetallbase wird die Lösung abgelassen. Danach verdampft man das restliche Lösemittel, das den so im Reaktor behandelten Feststoffen anhaftet und erhält auf diese Weise als Katalysator einen mit einer Alkalimetall- und/oder Erdalkalimetallbase dotierten porösen Träger. Hochsiedende Lösemittel wie Glykole oder Polyalkylenglykole, die den Katalysator nach dem Ablassen des Lösemittels noch benetzen, werden vorzugsweise durch Erhitzen des Katalysators auf Temperaturen bis zu 270 °C unter vermindertem Druck, z. B. bei 10 bis 100 mbar, entfernt.

Der in den Rohrreaktor eingebrachte Feststoff wird vorzugsweise mit einer wässrigen Lösung von Natriumcarbonat oder Kaliumcarbonat behandelt. Ein besonders bevorzugt eingesetzter Katalysator wird durch Behandlung von α-Aluminiumoxid mit einer wäßrigen Lösung von Natriumcarbonat oder Kaliumcarbonat hergestellt. Das Trocknen der imprägnierten Feststoffe erfolgt zunächst beispielsweise dadurch, dass man einen Inertgasstrom durch den Reaktor leitet und die Temperatur des Inertgasstroms kontinuierlich oder schrittweise erhöht. Man kann jedoch auch den Reaktor von außen beheizen und zusätzlich einen Inertgasstrom durch die Katalysatorfüllung leiten. Als Inertgase kommen beispielsweise überhitzter Wasserdampf, Luft oder Stickstoff in Betracht. Wenn man den Katalysator mit einer wässrigen Lösung einer Alkalimetall- oder Erdalkalirnetallbase behandelt hat, wird er zunächst bei einer Temperatur bis zu 200 °C, vorzugsweise in dem Temperaturbereich von 160 bis 195 °C getrocknet. Der Trocknungsprozess ist beendet, wenn im Abgas praktisch kein Wasserdampf mehr nachweisbar ist. Nach dem Trocknen werden die imprägnierten Feststoffe, d.h. die Katalysatoren, zur Aktivierung auf eine Temperatur von mindestens 380 °C, meistens mindestens 430 °C, insbesondere mindestens 450 °C erhitzt. Vorzugsweise wird die Aktivierung des Katalysators in dem Temperaturbereich von 450 bis 550 °C vorgenommen.

Die Aktivierung des Katalysators erfolgt in der Regel bei einer Temperatur, die mindestens 10 °C oberhalb der Temperatur liegt, bei der die Pyrolyse der Verbindungen der Formel I vorgenommen wird. Vorzugsweise erhitzt man den Katalysator nach dem Trocknen im Rohrreaktor auf eine Temperatur, die mindestens 50 °C oberhalb der Temperatur liegt, bei der anschließend die Pyrolyse durchgeführt wird. Das Tempern des Katalysators dauert beispielsweise 5 bis 72 Stunden.

Wenn man einen porösen Feststoff, insbesondere α-Aluminiumoxid, mit einer wässrigen Lösung von Natriumcarbonat und/oder Kaliumcarbonat oder einer wässrigen Lösung eines anderen Carbonats behandelt hat, kann man den Fortschritt des Temperns des Katalysators mit Hilfe einer Analyse des Abgases verfolgen. Beispielsweise tempert man den Katalysator im Rohrreaktor bei einer Temperatur von mindestens 380 °C und misst laufend den CO₂-Gehalt des Abgases. Das Tempern des Katalysators ist beendet, sobald im Abgas praktisch kein CO₂ mehr nachweisbar ist. Danach kühlt man den Katalysator auf die Temperatur ab, bei der anschließend die Pyrolyse durchgeführt wird.

Die Pyrolyse der N-Vinylcarbonsäureamide erfolgt unter vermindertem Druck. Dadurch wird eine unerwünschte Polymerisation und/oder Zersetzung der entstehenden Monomeren weitgehend zurückgedrängt bzw. verhindert. Bei der Pyrolyse liegen die Drücke in dem Bereich von beispielsweise 1 bis 500 mbar, vorzugsweise in dem Bereich von 5 bis 200 mbar. Die Temperaturen bei der Pyrolyse liegen z.B. in dem Temperaturbereich von 330 bis 750 °C, vorzugsweise 350 bis 550 °C. Geeignete Reaktionsbedingungen werden beispielsweise in der DE-C 1 224 304, der EP-A 0 184 074, Spalte 1, Zeile 51 bis Spalte 3, Zeile 53 und EP-A 0 184 694, Spalte 2, Zeile 34 bis Spalte 6 eingehend beschrieben. Die dort gemachten Angaben gelten entsprechend für erfindungsgemäße Verfahren. Die bei der Pyrolyse entstehenden Stoffe, die unter den Reaktionsbedingungen gasförmig sind, werden abgekühlt, so dass die jeweils gebildeten Monomeren kondensieren. Sie werden kontinuierlich oder absatzweise ausgeschleust, nach dem Druckausgleich mit der Atmosphäre von geringen Mengen an Cyanwasserstoff befreit und gereinigt. Der Cyanwasserstoff kann gegebenenfalls durch Abkühlen auf tiefe Temperaturen und Druckausgleich mit dem Atmosphärendruck als Flüssigkeit gewonnen werden. Aus sicherheitstechnischen Gründen ist jedoch die Chemisorption des Cyanwasserstoffs an Basen oder Carbonylverbindungen wie sie in den obengenannten EP-Anmeldungen beschrieben ist, bevorzugt. Pyrolyse und Chemisorption werden kontinuierlich durchgeführt.

Der nach dem erfindungsgemäßen Verfahren hergestellte, aktivierte Katalysator hat beispielsweise eine Standzeit von mindestens 12 Wochen. Sobald die Selektivität des Katalysators und/oder der Umsatz abnehmen, muss der Katalysator regeneriert werden. Beispielsweise regeneriert man den Katalysator, sobald der Umsatz auf einen Wert von 80 %, meistens 90 % gesunken ist. Hierbei geht man in der Regel zunächst so vor, dass man kohlenstoffhaltige Ablagerungen auf dem Katalysator in einem Inertgasstrom, wie überhitztem Wasserdampf, unter Zufuhr von Sauerstoff oxydiert. Falls eine solche Aktivierung des Katalysators nicht mehr möglich ist, weil die Alkalikonzentration auf dem Kontakt nicht mehr ausreicht, wird die Produktion von N-Vinylcarbonsäureamiden unterbrochen und der Katalysator im Rohrreaktor auf eine Temperatur abgekühlt, bei der dann die erfindungsgemäße Regenerierung des Katalysators durch Behandlung mit einer Lösung einer Alkalimetall- und/oder Erdalkalimetallbase erfolgt.

Wenn man beispielsweise den Reaktor mit Hilfe eines Salzbades beheizt, so kann man ihn zur Aktivierung des Katalysators z. B. auf eine Temperatur in dem Bereich von 200 bis 250 °C abkühlen und dann - sofern er für höhere Drücke ausgelegt ist - den Katalysator mit einer wässrigen Lösung einer Alkalimetall- und/oder Erdalkalimetallbase unter Druck behandeln ohne das Salzbad abzulassen. Allerdings wird das Salzbad dann ebenfalls auf eine Temperatur in dem genannten Bereich abgekühlt. Die Behandlung des Katalysators kann jedoch auch in dem Temperaturbereich von 200 bis 250 °C unter Atmosphärendruck erfolgen, sofern man ein Lösemittel oder ein Lösemittelgemisch für die Basen verwendet, das bei Normaldruck oberhalb von 250 °C, vorzugsweise oberhalb von 300 °C siedet. Nach der Behandlung mit einer Lösung einer Base wird das restliche Lösemittel, das den Katalysator benetzt, entfernt und der Katalysator im Rohrreaktor durch Erhitzen auf eine Temperatur von mindestens 380 °C aktiviert. Der Reaktor kann aber auch z.B. über ein anderes Wärmeträgermedium, wie Öl oder direkt elektrisch oder auch mit heißen Gasen beheizt werden.

Wenn man die Behandlung des Katalysators mit einer Lösung einer geeigneten Base bei Temperaturen unterhalb des Siedepunkts des Lösemittels, z. B. in dem Bereich von 10 bis 80 °C vornehmen will, wird der Katalysator im Rohrreaktor auf eine Temperatur in diesem Bereich abgekühlt. Das Abkühlen kann in zwei Stufen durchgeführt werden, indem man den Katalysator im Reaktor zunächst auf eine Temperatur in dem Bereich von 200 bis 250 °C unter Atmosphärendruck abkühlt und ihn dann unter vermindertem Druck in einem Inertgasstrom auf eine Temperatur unterhalb von 100 °C abkühlt. Als Inertgas kann man beispielsweise Luft, Stickstoff oder Wasserdampf einsetzen. Beispielsweise kann man zum Trocknen des Katalysators Wasserdampf unter vermindertem Druck (z.B. bei 10 bis 700 mbar, vorzugsweise 20 bis 100 mbar, insbesondere bevorzugt 30 bis 50 mbar) durch den Reaktor leiten. Nach dem Druckausgleich mit der Atmosphäre und Imprägnieren des Katalysators mit einer Lösung einer Base, lässt man die Lösung der Base aus dem Rohrreaktor ab und entfernt das verbliebene Lösemittel durch Erhitzen des so behandelten Katalysators im Rohrreaktor. Zusätzlich kann man noch einen Inertgasstrom durch den Reaktor leiten und/oder verminderten Druck anwenden, z. B. Drücke von 10 bis 500 mbar. Um schließlich den Katalysator zu aktivieren, erhitzt man ihn im Rohrreaktor auf eine Temperatur von mindestens 380 °C.

Während der Katalysator nach dem Stand der Technik aus dem Rohrreaktor ausgebaut und in einer separaten Vorrichtung imprägniert und gegebenenfalls auch getempert werden muss, kann er nach dem erfindungsgemäßen Verfahren zur Aktivierung und Regenerierung im Reaktor verbleiben. Dadurch ergibt sich für die Produktion von N-Vinylcarbonsäureamiden eine deutliche Zeitersparnis gegenüber dem bekannten Vorgehen. Außerdem wird eine mechanische Zerstörung des Katalysators aufgrund von Ausbau- und Einbaumaßnahmen vermieden. Nach der Aktivierung hat der Katalysator sofort gute Umsatz- und Selektivitätswerte.

Das erfindungsgemäße Verfahren hat insbesondere technische Bedeutung für die Herstellung von N-Vinylformamid, das durch Pyrolyse der Verbindung der Formel I mit R = H und R¹ = H (N-Formylalaninnitril) erhältlich ist. Aus N-Vinylformamid werden z. B. Polymere hergestellt, die gegebenenfalls nach vollständiger oder partieller Hydrolyse als Fixiermittel, Flockungsmittel und Retentionsmittel bei der Herstellung von Papier verwendet werden.

### Beispiel

Bei der Herstellung von N-Vinylformamid nach dem Verfahren der EP-A 0 184 074 bildeten sich auf dem Katalysator und an den Innenwänden des Reaktors, der aus einem Reaktorrohr mit Nennweite 80 mm bestand, kohlenstoffhaltige Ablagerungen, die den Umsatz erniedrigten und zu einem Druckverlust in der Anlage führten. Der Katalysator bestand aus Aluminiumoxid, das mit Kaliumionen aktiviert war. Ebenso nahm die Aktivität durch Verlust von Kalium als Aktivkomponente im Katalysatormaterial ab. Zunächst wurden die kohlenstoffhaltigen Ablagerungen auf dem Katalysator und im Reaktor entfernt, indem bei einer Temperatur von 530 °C 200 l/h Stickstoff und 1 kg/h überhitzten Wasserdampf eingeleitet wurden und später unter kontrollierten Bedingungen Luft in den Gasstrom zugemischt wurde. Nach insgesamt 15 Stunden betrug der CO₂-Gehalt im Abgas 0,05 Vol.-%. Katalysator und Reaktor waren nach dieser Zeit fast vollständig von kohlenstoffhaltigen Ablagerungen befreit.

Daraufhin wurde die Reaktortemperatur auf 205°C abgesenkt und anschließend die Salzschmelze in einen temperierten Auffangbehälter abgelassen. Durch Überleiten von zunächst 1300 l/h kalter Luft, dann nachdem die Temperatur auf 160°C gefallen war mit 1 kg/h Wasserdampf wurde die Temperatur des Reaktors weiter reduziert. Bei Erreichen von 120°C wurde auf das Reaktorsystem Vakuum 100 mbar angelegt, das dann sukzessive auf 50 mbar (abs) reduziert wurde, dann auf ca. 30°C gesenkt wurde, und anschließend wurde der untenliegende Reaktorablauf verschlossen. Der Reaktor wurde soweit mit einer 39,9 Gew.-%igen Kaliumcarbonat enthaltenden wässrigen Lösung befüllt, bis alle Katalysatorkörper bedeckt waren. Diese Lösung verblieb für 9 Stunden im Reaktor und wurde dann abgelassen.

Der Katalysator wurde anschließend durch Überleiten von 1300l/h mittels eines externen Lufterhitzers auf 230 °C erhitzter Luft getrocknet und auf 195 °C erhitzt. Daraufhin wurde der Wärmeträgerraum des Reaktor wieder mit der Salzschmelze befüllt und weiter aufgeheizt. Dabei wurde ein Strom von 1300l/h Luft über den Katalysator geleitet und die Kohlendioxidkonzentration gemessen. Nach anfänglichem Ansteigen der Konzentration ab einer Temperatur von ca. 350°C fiel sie bei weiterem Erhitzen wieder auf den ursprünglichen Blindwert von 0,05 Vol.-% ab. Als der Reaktor eine Temperatur von 550°C erreicht hatte wurde das Erhitzten beendet und der Reaktor auf 330°C abgekühlt. Der Druck im Reaktor wurde dann auf 10 mbar erniedrigt. Man leitete dann stündlich 2 kg Formylalaninnitril in Dampfform durch den Rohrreaktor. Das entstandene Reaktionsgas wurde mittels gekühltem flüssigem Reaktionsprodukt gequencht, wobei das aus dem Formylalaninnitril gebildete N-Vinylformamid kondensiert wurde. Es wurde kontinuierlich ausgeschleust und durch Destillation gereinigt. Der verbleibende Gasanteil, der im Wesentlichen aus Cyanwasserstoff bestand, wurde im Vakuum durch Acetaldehyd geleitet, um Cyanwasserstoff mit Hilfe einer Chemisorption zu entfernen.

## Patentansprüche

1. Verfahren zur Herstellung von N-Vinylcarbonsäureamiden durch Pyrolyse einer Verbindung der Formel 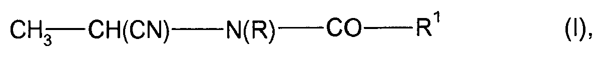 in der R und R¹ für H oder C₁- bis C₆-Alkyl stehen,
in Gegenwart von Feststoffen, die mit Alkalimetall- oder Erdalkalimetallionen dotiert sind, unter vermindertem Druck bei einer Temperatur von 330 bis 750 °C unter Abspaltung von Cyanwasserstoff, Abkühlen, Trennen und Isolieren der Reaktionsprodukte, **dadurch gekennzeichnet, dass** man einen in einem Rohrreaktor angeordneten Feststoff bei einer Temperatur in dem Bereich von 0 bis 250 °C mit einer Lösung einer Alkalimetall- und/oder Erdalkalimetallbase behandelt, die Lösung ablässt, das restliche Lösemittel, das dem so erhältlichen Katalysator anhaftet, verdampft und ihn danach zur Aktivierung auf eine Temperatur von mindestens 380 °C erhitzt.

2. Verfahren nach Anspruch 1, **dadurch gekennzeichnet, dass** man den Katalysator nach dem Verdampfen des Lösemittels im Rohrreaktor auf eine Temperatur erhitzt, die mindestens 10 °C oberhalb der Temperatur liegt, bei der anschließend die Pyrolyse durchgeführt wird.

3. Verfahren nach Anspruch 1 oder 2, **dadurch gekennzeichnet, dass** man den Katalysator nach dem Verdampfen des Lösemittels im Rohrreaktor auf eine Temperatur erhitzt, die mindestens 50 °C oberhalb der Temperatur liegt, bei der anschließend die Pyrolyse durchgeführt wird.

4. Verfahren nach einem der Ansprüche 1 bis 3, **dadurch gekennzeichnet, dass** man als Katalysator einen mit einer Alkalimetall- und/oder Erdalkalimetallbase dotierten porösen Träger verwendet.

5. Verfahren nach einem der Ansprüche 1 bis 4, **dadurch gekennzeichnet, dass** man den Feststoff im Rohrreaktor mit einer wässrigen Lösung von Natriumcarbonat oder Kaliumcarbonat behandelt.

6. Verfahren nach Anspruch 5, **dadurch gekennzeichnet, dass** man den Katalysator im Rohrreaktor bei einer Temperatur von mindestens 380 °C tempert, wobei man den CO₂-Gehalt des Abgases laufend misst und das Tempern beendet, sobald im Abgas praktisch kein CO₂ mehr nachweisbar ist, und danach den Katalysator auf die Temperatur abkühlt, bei der anschließend die Pyrolyse durchgeführt wird.

7. Verfahren nach einem der Ansprüche 1 bis 4 und 6, **dadurch gekennzeichnet, dass** man die Pyrolyse bei einer Temperatur in dem Bereich von 350 bis 550 °C durchführt.

8. Verfahren nach einem der Ansprüche 1 bis 7, **dadurch gekennzeichnet, dass** man als Verbindung der Formel I N-Formylalaninnitril zur Herstellung von N-Vinylformamid einsetzt.

## Claims

1. A process for the preparation of N-vinylcarboxamides by pyrolysis of a compound of the formula 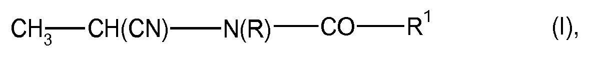 in which R and R¹ are H or C₁- to C₆-alkyl,
in the presence of solids which are doped with alkali metal or alkaline earth metal ions, under reduced pressure at a temperature of from 330 to 750°C with elimination of hydrogen cyanide, cooling, separation and isolation of the reaction products, wherein a solid arranged in a tubular reactor is treated with a solution of an alkali metal and/or alkaline earth metal base at a temperature in the range from 0 to 250°C, the solution is discharged, the remaining solvent which adheres to the catalyst thus obtained is evaporated and said catalyst is then heated to a temperature of at least 380°C for activation.

2. The process according to claim 1, wherein, after evaporation of the solvent, the catalyst is heated in the tubular reactor to a temperature which is at least 10°C above the temperature at which the pyrolysis is subsequently carried out.

3. The process according to claim 1 or 2, wherein, after evaporation of the solvent, the catalyst is heated in the tubular reactor to a temperature which is at least 50°C above the temperature at which the pyrolysis is subsequently carried out.

4. The process according to any of claims 1 to 3, wherein the catalyst used is a porous support doped with an alkali metal and/or alkaline earth metal base.

5. The process according to any of claims 1 to 4, wherein the solid is treated in the tubular reactor with an aqueous solution of sodium carbonate or potassium carbonate.

6. The process according to claim 5, wherein the catalyst is heated in the tubular reactor at a temperature of at least 380°C, the CO₂ content of the exit gas being continuously measured and the heating terminated as soon as virtually no more CO₂ is detectable in the exit gas, and the catalyst is then cooled to the temperature at which the pyrolysis is subsequently carried out.

7. The process according to any of claims 1 to 4 and 6, wherein the pyrolysis is carried out at a temperature in the range from 350 to 550°C.

8. The process according to any of claims 1 to 7, wherein N-formylalaninenitrile is used as the compound of the formula I for the preparation of N-vinylformamide.

## Revendications

1. Procédé pour la préparation d'amides d'acide N-vinylcarboxylique par pyrolyse d'un composé de formule 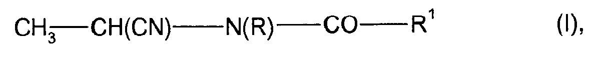 dans laquelle R et R¹ représentent H ou C₁-C₆-alkyle,
en présence de solides qui sont dopés par des ions de métal alcalin ou alcalino-terreux, sous pression réduite à une température de 330 à 750°C avec dissociation de cyanure d'hydrogène, refroidissement, séparation et isolement des produits de réaction, **caractérisé en ce qu'**on traite un solide disposé dans un réacteur tubulaire à une température dans la plage de 0 à 250°C avec une solution d'une base de métal alcalin et/ou de métal alcalino-terreux, on laisse partir la solution, on évapore le solvant résiduel qui adhère au catalyseur pouvant ainsi être obtenu et on chauffe celui-ci ensuite à une température d'au moins 380°C en vue de l'activation.

2. Procédé selon la revendication 1, **caractérisé en ce qu'**on chauffe le catalyseur après l'évaporation du solvant dans le réacteur tubulaire à une température qui est supérieure d'au moins 10°C à la température à laquelle la pyrolyse est ensuite réalisée.

3. Procédé selon la revendication 1 ou 2, **caractérisé en ce qu'**on chauffe le catalyseur après l'évaporation du solvant dans le réacteur tubulaire à une température qui est supérieure d'au moins 50°C à la température à laquelle la pyrolyse est ensuite réalisée.

4. Procédé selon l'une quelconque des revendications 1 à 3, **caractérisé en ce qu'**on utilise, comme catalyseur, un support poreux dopé d'une base de métal alcalin et/ou de métal alcalino-terreux.

5. Procédé selon l'une quelconque des revendications 1 à 4, **caractérisé en ce qu'**on traite le solide dans le réacteur tubulaire avec une solution aqueuse de carbonate de sodium ou de carbonate de potassium.

6. Procédé selon la revendication 5, **caractérisé en ce qu'**on traite thermiquement le catalyseur dans le réacteur tubulaire à une température d'au moins 380°C, la teneur en CO₂ de l'effluent gazeux étant mesurée en continu et le traitement thermique étant arrêté dès qu'on ne détecte pratiquement plus de CO₂ dans l'effluent gazeux, puis on refroidit le catalyseur à la température à laquelle la pyrolyse est ensuite réalisée.

7. Procédé selon l'une quelconque des revendications 1 à 4 et 6, **caractérisé en ce qu'**on réalise la pyrolyse à une température dans la plage de 350 à 550°C.

8. Procédé selon l'une quelconque des revendications 1 à 7, **caractérisé en ce qu'**on utilise, comme composé de formule I, du N-formylalaninenitrile pour la préparation de N-vinylformamide.
